# EUROPÄISCHE PATENTSCHRIFT

(11) **EP 0 913 199 B1**
(45) Veröffentlichungstag und Bekanntmachung des Hinweises auf die Patenterteilung: **15.01.2003**
(21) Anmeldenummer: 98118854.3
(22) Anmeldetag: 06.10.1998
(51) Int. Cl.: B01L 7/02, C12M 1/00

(54) **Einrichtung zur Befeuchtung des Nutzraumes eines Klimaschrankes**
Device for humidifying the working space of an incubator
Dispositif pour humidifier l'espace utile d'un incubateur

(30) Priorität: 28.10.1997 DE 19747498
(43) Veröffentlichungstag der Anmeldung: 06.05.1999
(73) Patentinhaber: KENDRO Laboratory Products GmbH, 63450 Hanau (DE)
(72) Erfinder: Maresch, Lothar, 63776 Mömbris (DE); Hessler, Egon, 63594 Hasselroth (DE)
(74) Vertreter: Lang, Friedrich

(56) Entgegenhaltungen:
- DE-B- 1 027 368
- DE-C- 3 815 528
- DE-U- 7 633 120
- PATENT ABSTRACTS OF JAPAN vol. 1995, no. 03, 28. April 1995 (1995-04-28) & JP 06 347107 A (HITACHI LTD;OTHERS: 01), 20. Dezember 1994 (1994-12-20)

## Beschreibung

Die Erfindung betrifft eine Einrichtung zur Befeuchtung des Nutzraumes eines Klimaschrankes, insbesondere eines Begasungsbrutschrankes, nach dem Oberbegriff des Anspruchs 1.

Um die Luft im Nutzraum von Klima- oder Begasungsbrutschränken zu befeuchten, werden verschiedene Arten von Befeuchtungseinrichtungen eingesetzt. Gemäß der einen Art wird Wasser aus einem Wasservorrat außerhalb des Nutzraumes verdampft und das Aerosol dem Nutzraum zugeführt. Eine andere Art, die Nutzraumluft zu befeuchten, besteht darin, einen Wasserbehälter direkt im Nutzraum anzuordnen und dort zu verdunsten. Überlicherweise wird hierzu eine separat entnehmbare Wasserwanne auf den Boden des Nutzraums angeordnet und mit einer geeigneten Heizvorrichtung, die direkt im Wasserbad oder in unmittelbarer Nähe des Wasserbades angeordnet sein kann, beheizt, oder der Nutzraum ist mit einem wannenförmigen Bodenbereich ausgestaltet.
Eine dem letztgenannten Prinzip entsprechende Befeuchtungs- bzw. Wasserdampfsterilisiereinrichtung ist beispielsweise aus DE-AS 10 27 368 bekannt. Hierbei weist ein Sterilisierschrank einen Nutzraum mit einem als Wanne ausgebildeten Bodenbereich auf, der mit zu verdampfendem Wasser gefüllt ist, wobei das Wasser über ein Zu- und Ableitungssystem mit einem externen Wasservorratsbehälter in Verbindung steht. Zur Tür des Sterilisierschrankes hin ist die Wanne durch einen wandartigen Steg begrenzt.

Die DE GM 7633120 offenbart einen Klimaschrank mit temperatur- und feuchtegeregelter Luft, der einen Nutzraum mit einer waagerechten Bodenfläche zur Aufnahme von Prüflingen und eine unterhalb des Nutzraumes angeordnete Verdunstungsfläche mit Neigung zu einem Ablauf hin aufweist. Auf die Verdunstungsfläche wird aus einem separaten Vorratsbehälter Wasser aufgebracht, das über ein Berieselungsrohr als laminar fließender Wasserstrom ausgebildet wird, über welchen vorbeistreichende Luft befeuchtet wird. Der über der Verdunstungsfläche angeordnete Nutzraum ist von einem Innengehäuse umschlossen. Der Klimaschrank selbst ist von einem doppelwandigen Außengehäuse umgeben, wobei zwischen der Innen- und Außenwandung eine Isolierung angeordnet ist und die Innenwandung die Verdunstungsfläche bildet. Der Aufbau beinhaltet demnach den Nutzraum, ein Innen- und ein Außengehäuse und ist dadurch aufwendig und schlecht reinigbar.

Weiterhin ist aus DE 38 15 528 ein Begasungsbrutschrank bekannt, der mit einer mit Wasser gefüllten Wanne am Boden des Nutzraumes oder einem als Wanne ausgebildeten Bodenbereich des Nutzraumes ausgestattet ist, was zur Befeuchtung des Nutzraumes dienen soll. Wird eine eingesetzte, rechteckige Wanne verwendet, so liegt diese flächig auf dem Boden des Nutzraumes auf und ist an allen vier Seiten durch senkrecht stehende Seitenwände begrenzt, die das Auslaufen des Wassers verhindern. Im Falle der wannenförmigen Ausbildung der Bodenfläche des Nutzraumes ist im unteren Teil der Öffnung des Nutzraumes des Begasungsbrutschrankes eine Querleiste angeordnet. Eine Reinigung des Wasserbehälters im Bodenbereich des Nutzraumes, insbesondere im vorderen, zur Öffnung des Nutzraumes hin ausgerichteten Bereich wird durch diese Konstruktion stark erschwert, da durch diese vordere Querleiste der unmittelbar dahinter liegende Bereich zur Inspektion und für die Einführung von Reinigungsmaterial, wie beispielsweise Reinigungstücher, nur schlecht zugänglich ist. Bei einer separaten, entnehmbaren Wanne mit waagrechtem Boden und rundum senkrechten Seitenwänden kommt außerdem als weiterer Nachteil die mögliche Kondensatbildung außerhalb der Wanne hinzu. Gerade im vorderen Bereich des Nutzraumes in Nähe der Öffnung wird von dem bis in diesen Bereich anstehenden Wasserbad die Feuchte der bei Öffnung des Nutzraumes eindringenden Außenluft außen an den Seitenwänden der Wanne die Kondensatbildung angeregt. Die sogenannte Feuchteerholzeit, die nach Öffnen des Begasungsbrutschrankes benötigt wird bis sich erneut stabile Feuchtewerte eingestellt haben, ist weiterhin bei einer Befeuchtungseinrichtung mit einer entnehmbaren Wanne mit verhältnismäßig schlechtem Wärmeübertragung von der Heizvorrichtung zum Wasserbad erheblich länger als bei einem Nutzraum mit intergriertem, wannenförmigen Bodenbereich.

Ausgehend von dem vorstehend aufgezeigten Stand der Technik liegt der vorliegenden Erfindung die Aufgabe zugrunde, eine Einrichtung zur Befeuchtung des Nutzraumes eines Klimaschrankes, insbesondere eines Begasungsbrutschrankes anzugeben, die zur Verringerung der Kontaminationsgefahr eine optimale Reinigung des Wasserbehälters zuläßt, die die Gefahr einer Kondensatbildung außerhalb des Wasserbehälters ausschließt oder zumindest verringert und die kostengünstig herstellbar ist.

Die Aufgabe wird dadurch gelöst, daß der Nutzraum mindestens im Bereich der Öffnung des Nutzraumes eine geneigte Bodenfläche aufweist, wobei der höhergelegene Bereich der Bodenfläche zur Öffnung des Nutzraumes hin, ohne durch eine Seitenwand begrenzt zu werden, ausgerichtet ist, der tiefergelegene Bereich der Bodenfläche jedoch zur Rückwand des Nutzraumes hin ausgerichtet ist oder direkt an die Rückwand anschließt.
Durch diese Konstruktion läßt sich die Befeuchtungseinrichtung von der Öffnung des Nutzraumes her leicht reinigen, da das Reinigungsmaterial auf der geneigten, im Nutzraum des Klima- oder Begasungsbrutschrankes nach hinten abfallenden Bodenfläche geführt werden kann. Durch den Wegfall einer vorderen, senkrecht stehenden Seitenwand oder Querleiste können Verunreinigungen auch am vorderen Teil der erfindungsgemäßen Befeuchtungseinrichtung sofort erkannt werden und leicht entfernt werden. Die Tiefe des Wasserbades nimmt in Richtung der Rückwand des Nutzraumes aufgrund der Schräge des Nutzraumbodens zu. Je nach Füllmenge wird daher im vorderen, zur Öffnung des Nutzraumes hin ausgerichteten Bereich der Befeuchtungseinrichtung gar kein oder nur wenige Millimeter tief Wasser stehen. Der für die Kondensatbildung kälteste Bereich des Nutzraumes liegt circa 10 Zentimeter oberhalb des Wasserbades an der Rückwand des Nutzraumes, so daß kondensierendes Wasser, beispielsweise aus der beim Öffnen des Nutzraumes eindringenden Außenluft sich bevorzugt dort niederschlägt und sofort von dort in das Wasserbad abfließt. Für den Hersteller von Klima- und Begasungsbrutschränken ergibt sich durch die Erfindung weiterhin ein Kostenvorteil durch den Wegfall der sonst üblichen vorderen, senkrecht stehenden Seitenwand bzw. Querleiste im unteren Bereich der Öffnung des Nutzraumes.

Zweckmäßigerweise weist die schräge Bodenfläche des Nutzraumes eines Klima- oder Begasungsbrutschrankes eine Neigung von 2° bis 10° auf.

Um einen Richtwert zur Wasser-Füllmenge der Befeuchtungseinrichtung zu erhalten, weist die geneigte Bodenfläche des Nutzraumes in ihrem höher gelegenen Bereich Markierungen auf. Die Markierungen können beispielsweise Eingravierungen oder Prägungen in der Bodenfläche sein oder kleine noppenartige Erhebungen oder Wülste auf der Bodenfläche oder an einer oder mehrerer der drei Seitenwände.

Weitere Einzelheiten und Merkmale der Erfindung ergeben sich aus der nachfolgenden Beschreibung des Ausführungsbeispiels anhand der Zeichnungen.

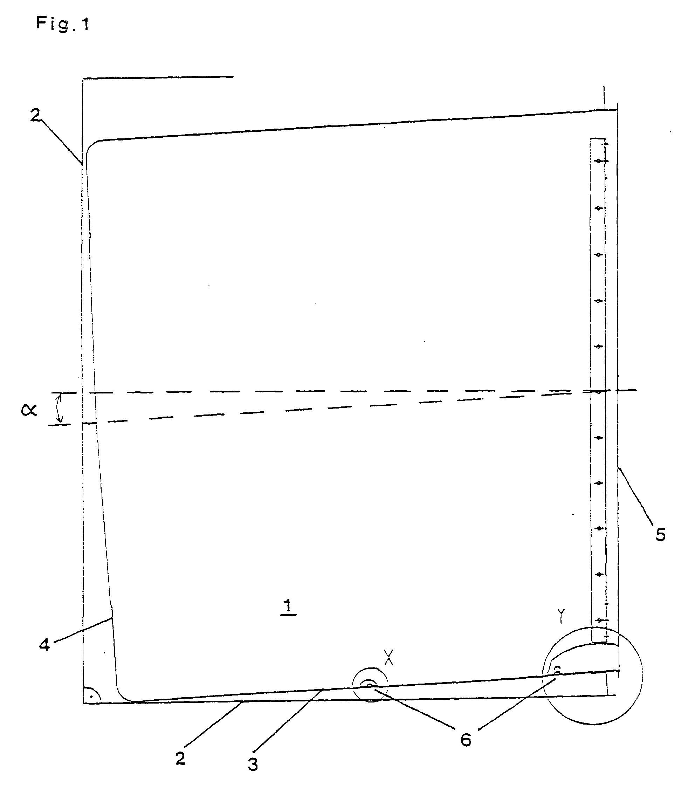

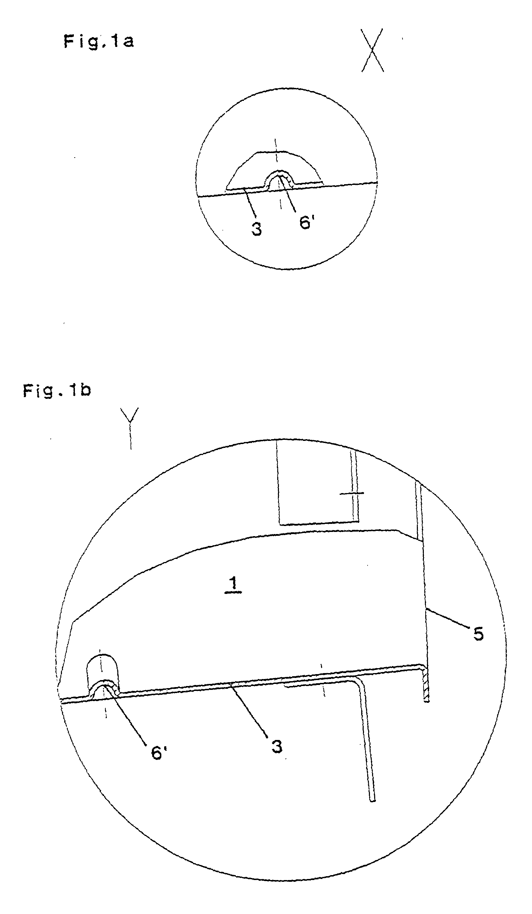

Dabei zeigt
- Figur 1: eine Seitenansicht eines Begasungsbrutschrank-Nutzraumes mit geneigter Bodenfläche;
- Figur 1a und 1b: die vergrößerten Ausschnitte X und Y aus Figur 1 mit wulstartigen Prägungen an der Bodenfläche.

Eine einfache Ausführungsform der erfindungsgemäßen Befeuchtungseinrichtung ist in Figur 1 dargestellt. In Seitenansicht wird gezeigt wie hierbei das Nutzraum- oder Innengehäuse, in an sich bekannter Ausführung, in Schrägstellung im Außengehäuse 2 eines Brutschrankes eingesetzt ist, der beispielsweise für medlzinisch/biologische Untersuchungen Verwendung findet. Der untere Bereich des Nutzraumes 1 dient dabei zur Aufnahme von Wasser im Sinne einer Wanne mit geneigter Bodenfläche 3, wobei die Neigung in Richtung der Rückwand 4 des Nutzraumes 1 bzw. des Brutschrankes erfolgt. Der Neigungswinkel α beträgt in diesem Fall 3 °, was bei den Innenabmessungen des Nutzraumes 1 von 470 mm (Breite), 530 mm (Tiefe), 607 mm (Höhe) ein maximale Wasserfüllmenge von 3 Litern ergibt. Die zur Förderung der Wasserverdunstung nötigen Heizelemente sind unterhalb der Bodenfläche 3 und im unteren Bereich, außerhalb des Nutzraumes 1 angeordnet. Die geneigte Bodenfläche 3 ist im vorderen, zur Öffnung 5 des Nutzraumes 1 hin ausgerichteten Bereich nicht durch eine senkrecht stehende Seitenwand oder Querleiste begrenzt, so daß sie von der Öffnung 5 des Nutzraumes 1 aus frei zugänglich und einsehbar ist. Für eine Reinigung verbleiben auf diese Art keine "toten Winkel". Die Bildung von Kondenswasser außerhalb dieser Befeuchtungseinrichtung ist außerdem praktisch nicht möglich, da sich eventuell aus der Außenluft (beim Öffnen des Nutzraumes) stammendes Wasser nur am Wasserbad selbst oder an der Rückwand 4 des Nutzraumes 1 kurz oberhalb des Wasserbades niederschlagen wird und nicht etwa außen an einer Querleiste. Um dem Bedienpersonal eine Orientierung für die Füllmenge des Wasser zu geben, weist die geneigte Bodenfläche 4 etwa in der Mitte und in Nähe der Öffnung 5 des Nutzraumes 1 Markierungen 6 in Form einer wulstartigen Prägung 6' (Höhe ca. 3 bis 5 mm) auf. Die wulstartige Prägung 6' kann über der gesamten Nutzraum- oder Wannenbreite ausgebildet sein. In den Figuren 1a und 1b sind diese Prägungen 6' als Ausschnittsvergrößerungen X bzw. Y, die der Originalgröße entsprechen, dargestellt.

## Patentansprüche

1. Einrichtung zur Befeuchtung des Nutzraumes (1) eines Klimaschrankes, wobei der Nutzraum (1) selbst eine wannenförmige, ein Wasserbad aufnehmende Bodenfläche (3;3') und Seitenwände (8; 8'; 8") aufweist,
**dadurch gekennzeichnet,**
**dass** der Nutzraum (1) mindestens im Bereich der Öffnung (5) des Nutzraumes (1) eine zur Rückwand (4) des Nutzraums hin geneigte Bodenfläche (3;3') aufweist, und der höhergelegene Bereich der Bodenfläche (3;3') zur Öffnung (5) des Nutzraumes (1) hin ausgerichtet ist und bündig mit der Öffnung abschließt.

2. Einrichtung zur Befeuchtung des Nutzraumes (1) eines Klimaschrankes nach Anspruch 1,
**dadurch gekennzeichnet,**
**dass** die Neigung der Bodenfläche (3, 3') des Nutzraumes (1) im Bereich von 2° bis 10° liegt.

3. Einrichtung zur Befeuchtung des Nutzraumes (1) eines Klimaschrankes nach einem der vorhergehenden Ansprüche,
**dadurch gekennzeichnet,**
**dass** die geneigte Bodenfläche (3; 3') zur Kennzeichnung des Füllstandes des Wasserbades Markierungen (6, 6') aufweist.

4. Einrichtung zur Befeuchtung des Nutzraumes (1) eines Klimaschrankes nach einem der vorhergehenden Ansprüche,
**dadurch gekennzeichnet,**
**dass** sich die Neigung über die gesamte Bodenfläche (3; 3') erstreckt.

5. Einrichtung zur Befeuchtung des Nutzraumes (1) eines Brutschrankes nach Anspruch 4,
**dadurch gekennzeichnet,**
**dass** die Neigung der Bodenfläche (3; 3') dadurch erzeugt wird, dass ein an sich bekannter Nutzraum (1) mit einer Neigung in Richtung seiner Rückwand (4) in den Brutschrank eingesetzt ist.

## Revendications

1. Dispositif pour humidifier la chambre utile (1) d'une armoire climatique, la chambre utile (1) présentant elle-même une surface de sol (3 ; 3') en forme de bac recevant un bain d'eau, ainsi que des parois latérales (8 ; 8' ; 8"),
***caractérisé en ce que*** la chambre utile (1) présente, au moins au niveau de l'ouverture (5) de la chambre utile (1), une surface de sol (3 ; 3') inclinée en direction de la paroi arrière (4) de la chambre utile, et la partie supérieure de la surface de sol (3 ; 3') est orientée en direction de l'ouverture (5) de la chambre utile (1) et s'arrête en affleurement avec l'ouverture.

2. Dispositif pour humidifier la chambre utile (1) d'une armoire climatique selon la Revendication 1, ***caractérisé en ce que*** l'inclinaison de la surface de sol (3 ; 3') de la chambre utile (1) est comprise entre 2° et 10°.

3. Dispositif pour humidifier la chambre utile (1) d'une armoire climatique selon l'une quelconque des Revendications précédentes, ***caractérisé en ce que*** la surface de sol inclinée (3 ; 3') présente des repères (6, 6') pour indiquer le niveau de remplissage du bain d'eau.

4. Dispositif pour humidifier la chambre utile (1) d'une armoire climatique selon l'une quelconque des Revendications précédentes, ***caractérisé en ce que*** l'inclinaison s'étend sur toute la surface de sol (3 ; 3').

5. Dispositif pour humidifier la chambre utile (1) d'une armoire climatique selon la Revendication 4, ***caractérisé en ce que*** l'inclinaison de la surface de sol (3 ; 3') est obtenue par le fait qu'une chambre utile (1) connue en soi est insérée dans l'armoire climatique avec une inclinaison en direction de sa paroi arrière (4).

## Claims

1. Device for humidifying the useful space (1) of a climatic cabinet, the useful space (1) itself having a trough-shaped floor surface (3;3') holding a water bath, and having side walls (8;8';8")
**characterized in that**
the useful space (1) has at least at the area of the opening (5) of the useful space (1) a floor surface (3;3') which is inclined so as to slope down toward the backwall (4) of the useful space, and that the higher area of the floor surface (3;3) is oriented toward the opening (5) of the useful space (1) and is flush with the opening.

2. Device for humidifying the useful space (1) of a climatic cabinet according to claim 1,,
**characterized in that**
the floor surface (3;3') of the useful space (1) is inclined at an angle in a range between 2° to 10°.

3. Device for humidifying the useful space (1) of a climatic cabinet according to one of the preceding claims,
**characterized in that**
the inclined floor surface (3;3') has markings (6;6') to indicate the level of water in the water bath.

4. Device for humidifying the useful space (1) of a climatic cabinet according to one of the preceding claims 1 to 3,
**characterized in that**
the inclination is extending over the entire floor surface (3;3).

5. Device for humidifying the useful space (1) of an incubator according to claim 4,
**characterized in that**
the inclination of the floor surface (3;3') is achieved by inserting a known useful space (1) in the incubator, the useful space being inclined in the direction of its back wall.
